(19) Europäisches Patentamt
European Patent Office
Office européen des brevets

(11) Veröffentlichungsnummer: **0 095 682**
**B1**

# (12) EUROPÄISCHE PATENTSCHRIFT

(45) Veröffentlichungstag der Patentschrift: **26.08.87**

(51) Int. Cl.[4]: **C 07 G 17/00**, A 61 K 35/12, A 61 K 37/22

(21) Anmeldenummer: **83105027.3**

(22) Anmeldetag: **20.05.83**

(54) **Verfahren zur Herstellung zell- und geweberegenerierender Stoffe.**

(30) Priorität: **28.05.82 CH 3328/82**

(43) Veröffentlichungstag der Anmeldung:
**07.12.83 Patentblatt 83/49**

(45) Bekanntmachung des Hinweises auf die Patenterteilung:
**26.08.87 Patentblatt 87/35**

(84) Benannte Vertragsstaaten:
**AT BE CH DE FR GB IT LI LU NL SE**

(56) Entgegenhaltungen:
**EP - A - 0 038 511**
**FR - A - 2 117 884**
**FR - A - 2 195 425**
**US - A - 2 912 359**
**US - A - 3 672 954**

(73) Patentinhaber: **Solco Basel AG, Gellertstrasse 18, CH-4052 Basel (CH)**

(72) Erfinder: **Fraefel, Wolfgang, Prof., CH-1772 Grolley (CH)**
Erfinder: **Tschannen, Roland, Dr., Gerbergässlein 27, CH-4051 Basel (CH)**

(74) Vertreter: **Frossard, Michel, Dr. et al, A. Braun, Braun, Héritier, Eschmann AG, Patentanwälte Holbeinstrasse 36-38, CH-4051 Basel (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Es waren bereits verschiedene Verfahren bekannt, durch welche aus tierischen Organen therapeutisch wirksame Extrakte oder Stoffe gewonnen werden. Insbesondere wird nach FR-A 2 117 884 Milzgewebe zu einem Brei zerrieben, der Brei in eine kolloidale Suspension übergeführt und diese einer Dialyse unterworfen, so dass die Stoffe mit einem Molekulargewicht bis zu 5000 erhalten werden. Überaus wichtig dabei ist eine schonende, durch eine speziell für diesen Zweck entwickelte Mühle zu bewerkstelligende Zerkleinerung bis zur kolloidalen Grösse, damit die aus den Zellen freigesetzten Wirkstoffe nicht durch chemische oder biologische Einwirkung zersetzt oder verändert werden.

Gemäss FR-A 2 195 425 geht ein anderes Verfahren von tierischen Organen, wie Leber, Placenta, Nieren oder Milz, aus. Es wird das Organ schonend homogenisiert, das Homogenisat der Einwirkung von proteolytischen Enzymen, vorzugsweise Enzyme aus der Bauchspeicheldrüse, unterworfen und filtriert, das Filtrat bei saurem pH-Wert und erhöhter Temperatur behandelt und nach nochmaliger Filtration dialysiert. Durch die von aussen zugefügten Enzyme werden Proteine der Organe unter Bildung von wasserlöslichen Stoffen zersetzt. Das Verfahren ergibt Extrakte von hoher Konzentration an Wirkstoffen, ohne Verwendung von fremden Lösungsmitteln.

Es waren auch aus EP-A-38 511 gewisse Verbindungen bereits bekannt, welche in Organen und Körperflüssigkeiten von Säugetieren vorkommen bzw. daraus extrahiert werden können und die auf die Wundheilung eine beschleunigende Wirkung ausüben. Gemäss der erwähnten Veröffentlichung kommen den extrahierten Wirkstoffen insbesondere die Struktur eines Glykosteroides bzw. eines Glykosphingolipides zu.

Das Glykosteroid soll der folgenden Strukturformel entsprechen:

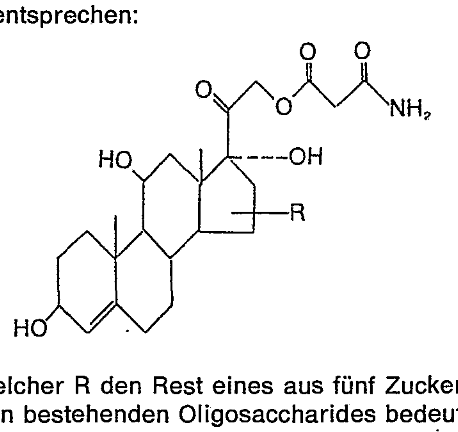

in welcher R den Rest eines aus fünf Zuckereinheiten bestehenden Oligosaccharides bedeutet.

Das Glykosphingolipid seinerseits soll die folgende Struktur aufweisen:

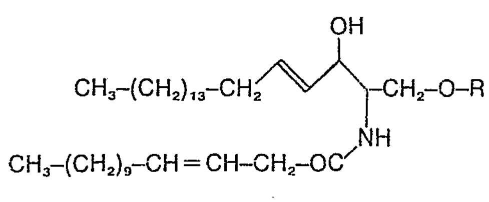

in welcher R den Rest eines aus fünf Zuckereinheiten bestehenden Oligosaccharides bedeutet.

Diese Verbindungen sind aus Organen, wie dem Herzen, der Lunge, den Muskeln und der Milz, und aus Körperflüssigkeiten, wie dem Blut, dem Plasma und dem Serum, isoliert worden. Säugetiere, welche die Verbindungen erzeugen, sind der Mensch, das Schwein, das Pferd, das Schaf und das Rind.

Das Herstellungsverfahren gemäss der erwähnten Patentanmeldung umfasst insbesondere eine Adsorption an Aktivkohle (Affinitätschromatographie) und Extraktion mit 10-30%iger Essigsäure, eine zweite Chromatographie auf einem Molekularsieb und Elution mit verdünnter Essigsäure, und eine dritte Chromatographie auf Kieselgel und Elution mit einem Gemisch von Äthanol und Methylenchlorid bzw. auf mit $C_8$-Ketten überzogenem Kieselgel und Elution mit Gemischen von Essigsäure, Äthanol und Wasser.

Es wurde nun überraschenderweise gefunden, dass man aus denselben Ausgangsmaterialien, welche oben angeführt werden, ebenfalls Glykolipide mit zell- und geweberegenerierenden Eigenschaften, jedoch in wesentlich höherer Ausbeute erhalten kann, wenn man das Ausgangsmaterial einer Autolyse und einer Dialyse gegen ein alkoholisch-wässriges Medium unterwirft. Dadurch bekommt man in der Tat ein Rohprodukt, in welchem die gewünschten Wirkstoffe in erstaunlich grösseren Mengen angereichert sind, als es nach dem bereits bekannten Verfahren der Fall war.

Es zeigte sich allerdings bei der Entwicklung des erfindungsgemässen Verfahrens, dass die zuvor erwähnten Glykosteroide bei der therapeutischen Anwendung eine ungenügende Beständigkeit aufweisen, um weiteres Interesse zu verdienen.

Ferner wurde auch gefunden, dass man aus dem erwähnten Rohprodukt die Glykolipide auf bedeutend einfacherem Wege isolieren kann, indem man sie (A) aus einer äthanolischen Lösung bei tiefer Temperatur, z. B. bei etwa –20 °C, ausfällt oder (B) mit einem mit Wasser nicht oder nur begrenzt mischbaren organischen Lösungsmittel extrahiert und das Produkt aus der Stufe (A) bzw. (B) an Kieselgel oder an einem Ionenaustauscher mit einem wenig polaren Lösungsmittel chromatographiert. Denkt man an die Aufwendigkeit der chromatographischen Trennungen, so wird aus der Notwendigkeit einer einzigen Chromatographie ein erheblicher Vorteil des neuen gegenüber dem bekannten Verfahren augenfällig, zumal wenn es um eine industrielle Anwendung gehen soll.

Überhaupt eignet sich das neue, in deutlichem Gegensatz zum älteren Verfahren, vorzüglich zur Durchführung in industriellem Massstabe, auch nicht zuletzt dadurch, dass die wesentlichen Stufen der Autolyse und Dialyse gleichzeitig und kontinuierlich, im Gegenstromverfahren, vorgenommen werden können.

Das erfindungsgemässe Verfahren ist dadurch gekennzeichnet, dass man das Blut oder Organhomogenisat zuerst mit einem kurzkettigen Alkohol und einem Bakteriostatikum versetzt und dann einer Autolyse während einer Zeitspanne von mehreren Stunden bis einigen Tagen bei einer Temperatur von 4 °C bis Raumtemperatur und einer Dialyse gegen ein Gemisch von Wasser und einem niederen aliphatischen Alkohol unterwirft, derart, dass die Stoffe mit einem Molekulargewicht unterhalb 10 000 sich in dem Dialysat befinden.

Im folgenden wird die Erfindung ausführlicher beschrieben.

Unter den oben erwähnten Ausgangsmaterialien werden defibriniertes Blut und Organhomogenisate von Säugetieren, wird Rinder- oder Kälberblut, vor allem letzteres, bevorzugt.

Es ist vorteilhaft für die Auflösung der während der Autolyse entstehenden Stoffe zuerst das Ausgangsmaterial mit einem kurzkettigen Alkohol, vorzugsweise Methanol oder Äthanol in einer Konzentration von 10 bis 30%, und mit einem Bakteriostatikum, vorzugsweise dem 4-Hydroxybenzoesäure-methylester oder 4-Hydroxybenzoesäure-propylester in einer Konzentration von 0,1 bis 0,0001%, zu versetzen. Die Autolyse kann dadurch erfolgen, dass man das Ausgangsmaterial während einer Zeitspanne von mehreren Stunden bis einigen Tagen, beispielsweise während drei Tagen, bei einer Temperatur von 4 °C bis Raumtemperatur sich selbst überlässt. Jedoch ist es vorteilhafter, die beiden Massnahmen gleichzeitig und kontinuierlich, im Gegenstromverfahren, durchzuführen, da durch die fortdauernde Entnahme der Autolyseprodukte das Gleichgewicht auf der Seite des Ausgangsmaterials ständig zugunsten der Autolysereaktion verschoben wird. Während der Autolyse werden intrazelluläre katabolische Enzyme, wie die Lysosomen, freigesetzt, z. B. die Kathepsine und andere Proteasen, ferner Nucleotidasen, Esterasen, Phosphatasen usw.

Im Dialysat finden sich die Stoffe mit einem Molekulargewicht unterhalb 10 000, darunter die gewünschten Glykolipide. Die Dialyse bewirkt also, gleichzeitig mit einer Beschleunigung der Autolyse, die Entfernung insbesondere jener Proteine, welche bei der therapeutischen Anwendung des Produktes zur Auslösung von allergischen Reaktionen führen können. Als Medium zur Aufnahme der Stoffe während der Dialyse sind Gemische von Wasser und niederen aliphatischen Alkoholen, beispielsweise Methanol und Äthanol, zweckmässig. Besonders geeignete Gemische bestehen aus Wasser und 10 bis 30 Vol.-% Äthanol, z. B. Wasser mit 15 Vol.-% Äthanol.

Gegebenenfalls wird das Rohprodukt für die nachfolgenden Isolierungs- und Trennungsmassnahmen intermediär geschützt. Es eignen sich hierzu ganz allgemein dieselben Schutzgruppen, welche von der Peptidchemie her für diesen Zweck bekannt sind. Durch selektive Einführung von Schutzgruppen werden also die funktionellen Gruppen der Wirkstoffe blockiert und die Wirkstoffe in eine für die Extraktion aus dem Reaktionsgemisch günstigere Form gebracht. Nach erfolgter Isolierung und Reinigung werden die Schutzgruppen abgespaltet. Beispiele von geeigneten Schutzgruppen und der Methoden für deren Abspaltung sind die Benzoylgruppe/Abspaltung durch verdünnte Alkalilauge; die Benzyloxycarbonylgruppe/Abspaltung durch Bromwasserstoff in Eisessig, Trifluoressigsäure oder anderen organischen Lösungsmitteln, Chlorwasserstoff in Äthanol, Trifluoressigsäure in der Wärme oder metallisches Natrium in flüssigem Ammoniak; die Benzyl- und die p-Toluolsulfonylgruppe/Abspaltung durch Natrium in flüssigem Ammoniak; die tert.-Butylgruppe/Abspaltung durch Chlorwasserstoff in organischen Lösungsmitteln, Bromwasserstoff in Eisessig, oder Trifluoressigsäure. Die tert.-Butylgruppe kann auf besonders elegante Art durch Säurekatalysierte Verätherung mit Isobuten eingeführt werden.

Zur Isolierung und Reinigung der Glykolipide aus dem erhaltenen Dialysat kann man (A) durch Ausfällung in der Kälte aus einer äthanolischen Lösung oder (B) durch Extraktion mit gewissen organischen, z. B. lipophilen, Lösungsmitteln, und danach Chromatographie an einem anorganischen Adsorptionsmittel oder an einem Ionenaustauscher vorgehen. Mit Vorteil kann zwischen der Extraktion (B) und der Chromatographie eine weitere Reinigung durch Ausfällung eingefügt werden.

Die Ausfällung (A) erfolgt aus einer äthanolischen Lösung bei einer Temperatur im Bereich von –15 bis –25 °C. Sie ergibt bessere Ausbeuten an Glykolipide, je höher die Endkonzentration an Äthanol ist. Zweckmässig gibt man dem Dialysat soviel wasserfreies Äthanol zu, dass davon eine Konzentration von mindestens 60 Vol.-% erreicht wird; die besten Ergebnisse werden bei einer Endkonzentration von 90 Vol.-% erhalten. Die gebildete äthanolische Lösung wird bei der erwähnten Temperatur mehrere Stunden lang stehen gelassen.

Die Abtrennung der festen Stoffe kann durch Filtration, Zentrifugation oder Sedimentation und Dekantierung erfolgen. Anschliessend wird das Produkt unter schonenden Bedingungen, d. h. bei einer Temperatur von höchstens 37 °C, getrocknet. Die Trocknung erfolgt durch Erwärmen oder im Vakuum oder durch beides gleichzeitig.

Für die Extraktion (B) wird das Dialysat entweder in getrockneter, fester Form oder in Form einer wässrigen Lösung oder einer Lösung in einem organischen Lösungsmittel eingesetzt. Zur Extraktion bedient man sich eines mit Wasser nicht mischbaren oder nur begrenzt mischbaren organischen Lösungsmittels oder Lösungsmittelgemisches. Es eignen sich insbesondere Äther, wie Diäthyläther und Tetrahydrofuran, Kohlenwasserstoffe, wie Pentan, Hexan, Petroläther und Benzol, halogenierte Kohlenwasserstoffe, wie Chloroform, Dichlormethan, Tetrachlorkohlenstoff, 1,2-Dichloräthan und Gemische von Chloroform und Methanol, Alkohole, wie Butanol und Amylalkohol, und Carbonsäureester, wie Äthylacetat und Butylacetat.

Durch diese Extraktion wird eine Auftrennung des Ausgangsproduktes in flüssige/flüssige Phasen oder in flüssige/feste Phasen, und damit eine

Auftrennung der Bestandteile in wenig polare oder apolare und polare oder ionische Substanzen erreicht. Die gewünschten Glykolipide gehen in die organische, weniger polare Phase über. Nach Abtrennung von der anderen flüssigen Phase oder von der festen Phase wird die organische Phase schonend getrocknet, wie oben bereits beschrieben wurde.

Auf die Extraktion folgt vorzugsweise eine Ausfällung der Glykolipide aufgrund ihrer jeweiligen Löslichkeit in einer wässrigen Lösung eines Metallsalzes und entweder Aceton, Diäthyläther, einem Gemisch der beiden, oder Äthanol bei einer Temperatur im Bereich von –15 bis –25 °C. Die wässrige Lösung enthält das Salz eines ein- bis fünfwertigen Metalls, vorzugsweise das Metallsalz einer anorganischen Säure. Es eignen sich insbesondere Magnesiumchlorid, Calciumchlorid, Natriumchlorid, Kaliumchlorid, Ammoniumsulfat, Natriumsulfat, Kaliumsulfat, Natriumnitrat, Dinatriumhydrogenphosphat und Natriumdihydrogenphosphat. Die wässrige Lösung kann eine beliebige Konzentration, bis hin zu Sättigung, aufweisen. In dieser Lösung wird nun der bei der Trocknung verbleibende Rückstand suspendiert oder gelöst.

Wenngleich Gemische von Aceton und Diäthyläther verwendet werden können, wird die Verwendung von Aceton oder Diäthyläther allein, oder von Äthanol in der Kälte, bevorzugt. Davon wird der zuvor gebildeten Suspension oder Lösung bis ein mehrfaches Volumen zugegeben und das Ganze wird gemischt. Die dabei entstehende Fällung enthält die Glykolipide; sie wird von der flüssigen Phase abgetrennt, was durch Filtration, Zentrifugation oder Sedimentation und Dekantierung erfolgen kann. Anschliessend wird das Produkt schonend getrocknet. Diese Arbeitsweise bildet eine erste Ausführungsform der Ausfällungsstufe.

Wenn die Löslichkeitsverhältnisse zur Ausfällung der Glykolipide aus dem Gemisch von Aceton oder Äther, oder Äthanol in der Kälte, und der wässrigen Metallsalzlösung erreicht sind, erfolgt die gewünschte Ausfällung. Gegebenenfalls enthält das durch Phasentrennung erhaltene Produkt eine ausreichende Konzentration an Salzen – insbesondere wenn es sich beim Ausgangsmaterial um Blut handelt, so dass die Ausfällung allein durch Zugabe von Aceton oder Äther, oder Äthanol in der Kälte, bewerkstelligt werden kann. Diese Arbeitsweise, bei welcher keine besondere Zugabe eines Metallsalzes oder einer Metallsalzlösung stattfindet, bildet eine zweite Ausführungsform der Ausfällungsstufe.

Gemäss einer dritten Ausführungsform kann man sogar die Extraktion und die Ausfällung in demselben Arbeitsgang durchführen. So kann man das Dialysat mit einem Gemisch des erwähnten mit Wasser nicht oder nur begrenzt mischbaren organischen Lösungsmittels und Aceton oder Äther in einem Volumenverhältnis zueinander und in einer Menge behandeln, welche sich nach dem Volumen und der Metallsalzkonzentration des Dialysates richten, und dadurch eine die Glykolipide enthaltende Fällung bewirken. Die Fällung wird von den flüssigen Phasen, wie oben angegeben, abgetrennt und schonend getrocknet.

In der letzten Verfahrensstufe wird das bei der Äthanolausfällung (A) oder der Extraktion (B) erhaltene Produkt chromatographisch gereinigt. Hierzu eignen sich anorganische Adsorptionsmittel, insbesondere Kieselgel oder Gemische von Kieselgel und einem Metalloxid oder einem Salz einer anorganischen Säure, beispielsweise die im Handel befindlichen Gemische von Kieselgel und Aluminiumoxid, Magnesiumoxid oder Magnesiumsulfat, oder aber Ionenaustauscher, insbesondere basische und saure Cellulosederivate wie Diäthylaminoäthancellulose, Carboxymethylcellulose oder Sulfopropylcellulose.

Das Produkt wird zuerst in einem wenig polaren organischen Lösungsmittel aufgelöst. Als Lösungsmittel eignen sich besonders Methanol, Gemische von Methanol und Chloroform und von Äthanol und Chloroform, ferner Äthylacetat und Gemische von Methanol, Chloroform und wenig Wasser. Die Lösung wird auf eine Chromatographiesäule gebracht, welche mit dem Adsorptionsmittel in dem gleichen Lösungsmittel oder einem anderen der oben angegebenen Lösungsmittel wasserfrei gefüllt worden ist. Zur Elution kann man dasselbe Lösungsmittel oder Lösungsmittelgemisch verwenden; man kann aber auch ein Lösungsmittelgemisch von zunehmender Polarität benutzen, was durch Anwendung eines linearen, konkaven oder konvexen oder eines Stufengradienten der quantitativen Zusammensetzung erreicht wird.

Die Fraktionen des Eluates können auf das Vorhandensein der Glykolipide sowie deren Gehalt und Reinheit nach verschiedenen Methoden geprüft werden. Es eignen sich dazu die im experimentellen Teil beschriebenen, für Glykolipide oder Glykosphingolipide charakteristischen Farbreaktionen und Färbemethoden. Man kann auch die Fraktionen auf mit Kieselgel, C-18-Alkan-derivatisiertem Kieselgel, Polyamid, Cellulose oder Polyacrylamid beschichteten Dünnschichtplatten oder -folien in einem geeigneten Fliessmittel weiter auftrennen. Die aufgetrennten Substanzen werden mit einem nicht wässrigen Lösungsmittel, z.B. einem Gemisch von Chloroform und Methanol, von der Platte oder Folie eluiert und mittels Dünnschichtchromatographie, Hydrolyse und Analyse der Hydrolyseprodukte, Gaschromatographie, Massenspektrometrie, Feststellung der Abheilungszeitverkürzung von Oberflächenläsionen an Säugetieren oder Beobachtung des Verhaltens von Zellkulturen, welchen diese Substanzen angeboten werden, untersucht. Die Ergebnisse zeigen, dass es sich bei den Wirkstoffen um Glycosphingolipide handelt.

Durch die nachfolgend beschriebenen pharmakologischen Versuche konnte nachgewiesen werden, dass die erhaltenen Wirkstoffe sowohl einen ausgeprägt proliferationsfördernden Einfluss auf vorgeschädigte Fibroblasten in vitro als auch einen regenerationsfördernden Effekt auf entsprechende Zellpopulationen in vivo haben. Dass es sich dabei nicht etwa um gewöhnliche mitotisch

wirkende Substanzen handelt, ist eindeutig, da normal gehaltene, gesunde Zellkulturen keine Veränderungen bei Zusatz der erwähnten Substanzen zeigen. Im Gegensatz dazu werden durch verschiedene schädigende Agenzien vorgeschädigte Kulturen mit einer abnormal tiefen Teilungsrate innert kurzer Zeit wieder auf eine normale Teilungsrate gebracht, die mit jener einer gesunden Kultur praktisch identisch ist.

Aufgrund der Tatsache, dass die Reparaturmechanismen geschädigter Zellpopulationen sowohl in vitro als auch in vivo durch die erfindungsgemäss hergestellten Stoffe angeregt werden, eignen sich diese für eine therapeutische Anwendung, speziell bei langsam oder schlecht heilenden Wunden bzw. Ulzerationen, deren Reparationsfähigkeit eingeschränkt ist.

Die fördernde Wirkung dieser Stoffe auf die Wundheilung kann durch folgende Tierversuche gezeigt werden.

Versuch 1

Narkotisierten Ratten werden die Fellhaare entfernt, und am Rumpf wird ihnen beidseitig durch Auflegen einer Messingscheibe mit einem Durchmesser von 2 cm und mit einer Temperatur von 270 °C während 30 Sekunden eine Verbrennungswunde gesetzt. Die Wirkstoffe werden in eine Gelée-Grundlage eingearbeitet, so dass ein 20%-Behandlungsgelée entsteht. Als Kontrolle wird Wasser oder physiologische Kochsalzlösung eingearbeitet. Je 10 Tiere mit 2 Wunden werden täglich zweimal mit dem Gelée lokal behandelt, und es wird die Dauer bis zur Endabheilung festgehalten. Die Substanz mit dem im Beispiel 2 beschriebenen $R_f$-Wert 0,65 ergibt gegenüber der Kontrollgruppe eine Verkürzung der Abheilungszeit bis zu 21%.

Versuch 2

An Minipigs werden dorsal je vier Verbrennungswunden wie in Versuch 1 beschrieben und mit einem Hohlzylinder-Bohrer 4 Stanzwunden mit einem Durchmesser von 2,5 cm gesetzt. Die Wirkstoffe werden bis zum Gehalt von 20% in eine Gelée-Grundlage eingearbeitet. Die Wunden werden zweimal täglich mit dem Gelée bestrichen, und die Dauer bis zur Endabheilung wird festgehalten. Die Substanz mit dem $R_f$-Wert 0,65 ergibt eine Verkürzung der Heilungsdauer um 18%.

Versuch 3

An Minipigs werden, wie in Versuch 1 beschrieben, Verbrennungswunden gesetzt. Nach 6, 12, 18 und 22 Tagen täglicher Wundbehandlung werden aus der Behandlungsgruppe Tiere entfernt und in Narkose getötet. Die Wunden werden ausgeschnitten, halbiert und in 4% gepuffertem Formalin fixiert. Diese Gewebestücke werden zu 4 µ dicken histologischen Paraffinschnitten verarbeitet. Folgende Parameter werden quantitativ erfasst:

1. Länge der epithelialisierten Wundoberfläche
2. Länge der nicht-epithelialisierten Wundoberfläche
3. Länge des Stratum basale der Epidermis
4. Fläche der neugebildeten Epidermis
5. Fläche von Haarfollikeln und Talgdrüsen

Die Auswertung der Parameter ergibt, dass die in den Beispielen 2 bis 6 beschriebene gereinigte Substanz die Bildung von Epithelzungen und deren Einwanderung zum Wundzentrum hin gegenüber den Kontrolltieren beschleunigt. Die beschleunigende Wirkung auf die Wundheilung zeigt sich vorwiegend in der frühen Phase der Ausbildung eines Stroma, des Stratum basale und der Papillen.

Versuch 4

An narkotisierten Ratten werden 1 cm breite und 5 mm tiefe Stanzwunden gesetzt. In diese Wundlöcher werden Viskose-Zellulose-Hohlzylinder-Schwämme eingesetzt. In die innere Aushöhlung der Hohlzylinder werden täglich 100 µl von gereinigter Substanz gegeben. 4, 10, 16 und 21 Tage nach der Implantation werden die Schwämme entnommen und auf den Gehalt an Hämoglobin, Desoxyribonukleinsäure und Hydroxyprolin untersucht. Die Wunden, welche mit den gereinigten Substanzen behandelt worden sind, haben an den Tagen 4 und 10 nach Implantation keinen höheren Gehalt an Hämoglobin, Desoxy-NS und Hydroxyprolin in den implantierten Schwämmen als die Kontrolltiere, an den Tagen 16 und 21 jedoch haben die Schwämme einen signifikant höheren Hämoglobin-, DNS- und Hydroxyprolin-Gehalt. Bezüglich der Methode, siehe J. Niinikoski und S. Renvall, Acta Chir. Scand. 145 (1979), 287–291.

Durch die gereinigte Substanz aus den Beispielen 2 bis 6 wird die Wundheilung in der frühen Ausbildung von Stroma und Stratum basale mit Kapillaren beschleunigt.

Versuch 5

Zellkulturen, z.B. wachsende Fibroblasten, können durch Auslassung des Natriumbicarbonats im Nährmedium in ihrem Wachstum gehemmt werden. Der Zusatz der in den Beispielen 2 bis 6 beschriebenen Substanzen zum Nährmedium führt dazu, dass die Zellen sich von dieser Hemmwirkung schneller erholen und die normale Teilungsrate deutlich früher wieder erreichen als die entsprechend gehemmten, aber unbehandelten Zellkulturen.

Die zahlenmässigen Angaben betr. Gemische von Lösungsmitteln in den Beispielen beziehen sich immer auf Volumina; z.B. bedeutet Äther : Äthanol $\doteq$ 3 : 1 ein Gemisch von 3 Vol.-teilen Äther und 1 Vol.-teil Äthanol.

Beispiel 1

Von Schlachttieren gewonnenes Kälberblut wird am Schlachtort sofort mit Äthanol bis zu einer Konzentration von 20% versetzt und mit den Bakteriostatika p-Hydroxybenzoesäuremethylester (Methylparaben) und p-Hydroxybenzoesäure-n-propylester (Propylparaben) bis zu einer Konzentration von jeweils 0,02% behandelt. Das Blut wird während 3 Tagen bei Raumtemperatur autolysiert,

wobei eine starke Hämolyse sowie ein teilweiser Abbau nichtstabiler Bestandteile stattfinden, und wobei Membrankomponenten aus den Membranen herausgelöst werden. Dieses Autolysat wird durch Dekantieren vom Sediment getrennt und der Überstand wird während 3 Tagen statisch oder dynamisch durch eine Membran mit der Ausschlussgrenze 10 000 dialysiert. Bei der dynamischen Dialyse werden Dialysat und Gegendialysat im Gegenstromverfahren während 3 Tagen durch die Dialysemembran gepumpt, so dass immer ein möglichst grosses Konzentrationsgefälle besteht.

Das resultierende Gegendialysat hat ein Trockengewicht von 5 bis 80 mg/ml und enthält wundheilungsfördernde Substanzen im Ausmasse bis zu 1 mg/ml. Nach dem Verfahren gemäss der eingangs erwähnten Europäischen Patentanmeldung werden entsprechende Glykosphingolipide im Ausmasse von 0,005 mg/ml erhalten.

Beispiel 2

2 Liter Kälberblut werden mit 300 ml Äthanol gemischt und während drei Tagen bei Raumtemperatur autolysiert. Das Autolysat wird durch einen Ultrafilter mit der Ausschlussgrenze 10 000 (Molekulargewicht) filtriert und dadurch von Zellenbruchstücken und grossen Proteinen getrennt. Das Filtrat wird mit 6 Litern Äthanol : Äther im Verhältnis 3 : 1 gemischt, wobei eine hauptsächlich aus Glycosphingolipiden bestehende Fällung entsteht. Die Fällung wird durch Zentrifugation mit 18 000 g während 30 Minuten sedimentiert und von der flüssigen Phase getrennt.

Die Fällung wird unter Vakuum bei 37 °C getrocknet und in 20 ml Chloroform : Methanol im Verhältnis 65 : 35 gelöst. Die gelösten Glykosphingolipide werden über eine Florisil-Säule (Markenname; Magnesiumsilicatgel, hochselektives Adsorbens der Firma Floridin Corp., Pittsburgh, PA, USA) mit dem Durchmesser 5,0 cm und der Länge 40 cm, die mit Chloroform : Methanol im Verhältnis 65 : 35 äquilibriert ist, chromatographisch aufgetrennt und dadurch restliche Phospholipide entfernt. Die Glykosphingolipide werden über einen Stufengradienten von je 2 Litern 35%, 50%, 75% Methanol in Chloroform und schliesslich mit 100% Methanol eluiert. Die bei den Elutionsstufen gewonnenen Substanzen werden dem in der Einleitung beschriebenen Wundheilungstest unterworfen und dabei wird festgestellt, dass die letzte, mit 100% Methanol eluierte Fraktion die Substanzen enthält, welche die Wundheilung beschleunigen.

Die in dieser Fraktion enthaltenen Substanzen werden auf präparative Kieselgel-Dünnschichtplatten mit 2 mm Schichtdicke aufgetragen und im Fliessmittel Chloroform : Methanol : 0,1% $CaCl_2$ in Wasser = 55 : 45 : 10 aufsteigend über eine Strekke von 18 cm chromatographiert. Die Platten werden nach der Chromatographie getrocknet und kurz in eine Kammer mit gesättigter Jod-Atmosphäre gestellt. Die dabei gelb anfärbenden Zonen werden markiert, nach Sublimation des Jods ausgekratzt und mit Chloroform : Methanol = 50 : 50 vom Kieselgel eluiert. Versuche zur Feststellung der Verkürzung der Abheilungszeit von Verbrennungswunden an Ratten verlaufen positiv mit der Fraktion, die im genannten dünnschichtchromatographischen Trennverfahren den $R_f$-Wert 0,65 hat.

Beispiel 3

2 Liter Gegendialysat von Beispiel 1 werden in einem Rotationsverdampfer oder durch Lyophilisation eingetrocknet und in 4 Litern Tetrahydrofuran : 0,01 M KCl im Verhältnis 4 : 1 aufgenommen. Unlösliches Material wird über einen Papierfilter abgetrennt und die klare Lösung wird in einem Rotationsverdampfer auf 500 ml eingeengt. Dazu wird 1 Liter Chloroform : Methanol im Verhältnis 2 : 1 gegeben, und die organische und wässrige Phasen werden durch Zugabe von 200 ml Wasser getrennt. Die obere Phase, welche die Glykolipide enthält, wird abgetrennt und zur Trockne eingeengt. Die trockene Substanz wird in 10 ml Chloroform : Methanol : Wasser = 90 : 10 : 0,2 gelöst und auf eine Bio-Sil A-Säule mit dem Durchmesser 1,5 cm und der Länge 120 cm, äquilibriert in Chloroform : Methanol : Wasser = 90 : 10 : 0,2, aufgetragen. Die Säule wird mit 1,5 Litern des gleichen Fliessmittels gewaschen und das Glykolipid wird danach mit 1 Liter Chloroform : Methanol : Wasser = 85 : 15 : 0,2 von der Säule eluiert. Die mit diesem Verfahren gereinigte Substanz ist ein Glykolipid, das die Abheilungszeit von Verbrennungswunden verkürzt.

Beispiel 4

2 Liter Gegendialysat von Beispiel 1 werden, wie in Beispiel 2 beschrieben, mit Äthanol : Äther im Verhältnis 3 : 1 extrahiert und die Fällung in 20 ml Chloroform : Methanol = 2 : 8 aufgenommen. Diäthylaminoäthyl-Sephadex A50 in der $Cl^-$-form wird durch Waschen mit 0,1 N Natronlauge und 1 N Essigsäure in die Acetat-Form gebracht, mit Methanol gewaschen und in eine Säule mit dem Durchmesser 2,5 cm und der Länge 20 cm gefüllt. Das in Chloroform : Methanol = 2 : 8 gelöste Material wird aufgetragen und die Säule mit je 200 ml 0,01 M Natriumacetat, 0,02 M Natriumacetat und 0,2 M Natriumacetat gewaschen. Dann wird das Glykolipid mit Chloroform : Methanol im Verhältnis 2 : 8 eluiert. Das nach diesem Verfahren eluierte Glykolipid verkürzt die Abheilungszeit von Verbrennungswunden.

Beispiel 5

200 ml Gegendialysat von Beispiel 1 werden in einem Rotationsverdampfer oder durch Lyophilisation zur Trockne eingeengt und in einem Exsikator während mindestens 3 Stunden über Phosphorentoxid getrocknet. Zum trockenen Material werden 15 ml frisch hergestellte 20%ige Lösung von Benzoesäureanhydrid in Pyridin zugegeben, danach 15 ml einer 10%igen Lösung von p-Dimethylaminopyridin in Pyridin. Das Gefäss wird dicht verschlossen und während 2 Stunden bei 37 °C inkubiert. Das Gefäss wird abgekühlt und der Inhalt über einen Filter oder durch Zentrifugation von ungelöstem Material getrennt.

Das Pyridin wird in einem Stickstoffstrom entfernt und das trockene Material in 30 ml Hexan aufgenommen. Das Hexan wird dreimal mit je 18 ml alkalischer Methanol-Wasser-Lösung, bestehend aus 0,4 g/100 ml Natriumcarbonat in Methanol : Wasser = 80 : 20, gewaschen. Die Hexanphase wird unter Stickstoff getrocknet und in 4% Äthylacetat in Hexan aufgenommen.

Das per-O-benzoylierte Glykolipid wird über eine Hochdruck-Flüssig-Chromatographie-Kieselgelsäule mit einem Gradienten von 4% bis 45% Äthylacetat in Hexan eluiert. Das nach diesem Verfahren isolierte per-O-benzoylierte Glykolipid wird zur Entfernung der Benzoylgruppen in 0,6 N Natronlauge während 1 Stunde bei 37 °C hydrolysiert. Das Hydrolysat wird mit 5 Volumen Aceton gemischt und das ausgefällte native Glykolipid abfiltriert oder zentrifugiert.

Das nach diesem Verfahren gereinigte Glykolipid verkürzt die Abheilungszeit von Verbrennungswunden.

Beispiel 6

Das Dialysat wird mit 9 Volumen Äthanol versetzt und während 30 Min. bei 85 °C gerührt. Der dabei entstehende Niederschlag wird über Filtrierpapier entfernt und die klare Lösung wird während drei Tagen bei –20 °C aufbewahrt. Die gesuchte Substanz fällt aus und sedimentiert während dieser Zeit. Der Überstand wird dekantiert und das Sediment wird in Chloroform : Methanol = 2 : 1 aufgenommen und auf eine Diäthylaminoäthyl-Sephacel-Säule in Acetatform aufgetragen. Die Säule wird mit Chloroform : Methanol = 2 : 1 eluiert und das Eluat gesammelt. Dieses wird in einem Rotationsverdampfer getrocknet und in Chloroform gelöst. Die Lösung wird auf eine Säule, welche mit aktiviertem Kieselgel in Chloroform gefüllt ist, aufgetragen. Zuerst wird mit Chloroform, dann mit Chloroform : Methanol = 9 : 1 gespült und diese Eluate werden verworfen. Danach wird mit Aceton : Methanol = 9 : 1 gespült und diese Fraktion enthält die gewünschten Glykolipide in reiner Form.

Kennzeichnung der Substanz

Die gemäss dem Beispiel 2 gereinigte Substanz wird auf Kieselgel-Dünnschichtplatten im Fliessmittel Methanol : Chloroform : 0,1% $CaCl_2$ in Wasser = 55 : 45 : 10 chromatographisch aufgetrennt.

Als Vergleichssubstanzen wird ein Gemisch der folgenden Substanzen simultan aufgetrennt: Asialogangliosid$_{M1}$, im folgenden mit Asialo-$GM_{M1}$ abgekürzt, Asialo-$G_{M2}$, Asialo-$G_{M3}$, Asialo-$G_{D1a}$, Cerebrosid und Psychosin.

Die Platten werden mit folgenden Methoden angefärbt:

1. 20 mg Kresylviolett in 1000 ml 1% Essigsäure; blauviolette Färbung.
2. 1% Diphenylamin in 2 ml Äthanol, 100 ml konz. Salzsäure und 80 ml Essigsäure; rote Färbung.
3. Zuerst 5,25% unterchlorige Säure in 40 ml Benzol und 5 ml Essigsäure besprühen, trocknen, dann 1% Benzidin in 50% Äthanol; blaue Färbung.
4. 0,1 g Orcinol in 1 ml 1% Eisen(III)-chlorid und 50 ml Wasser; braune Färbung.
5. Die Dünnschichtplatten werden in einer Kammer Jod-Dämpfen ausgesetzt. Die Vergleichssubstanzen sowie die zu analysierende Substanz mit dem $R_f$-Wert 0,65 werden gelb angefärbt.
6. Bei Verwendung von Dünnschichtplatten mit einem Fluoreszenzindikator für die Wellenlängen 254 nm und 366 nm wird die Fluoreszenz durch die zu analysierende Substanz mit dem $R_f$-Wert 0,65 nicht geschwächt, ebenso wie die Vergleichssubstanzen die Fluoreszierung der Platten nicht verändern.
7. 0,2% Naphthoresorcinol in Aceton und 10%ige Phosphorsäure als Spray-Reagens. Die behandelte Platte wird während 5 Minuten bei 90 °C erhitzt; blau-rote Färbung.
8. 1 g p-Ansidin in 100 ml 70% Äthanol; blaue Färbung.

Qualitative und quantitative Bestimmung

1 ml der gemäss dem Beispiel 2 gereinigten Substanz wird in einem Rotationsverdampfer unter Vakuum bei 37 °C oder durch Lyophilisation getrocknet. Die trockene Substanz wird in 0,8 ml 2M Schwefelsäure und 0,4 ml Dioxan aufgenommen und in Ampullen eingeschweisst. Die Proben werden während drei Stunden bei 95 °C hydrolysiert und danach durch Zugabe von 0,14 ml 10N Natronlauge neutralisiert. Das neutralisierte Hydrolysat wird mit 1,66 ml 0,2M Natriumborat-Puffer von pH 8,0 gepuffert und in einem Scheidetrichter mit 2,0 ml Diäthyläther während 5 Minuten extrahiert. Die untere Phase wird verworfen und die obere Ätherphase wird in einem Wasserbad bei 37 °C eingetrocknet. Die trockene Substanz wird in 3 ml Chloroform mit 0,0025% Fluorescamin-4-Phenylspiro-[furan-2(3H)-1′-phthalan]-3,3′-dion [M. Naoi, J. C. Lee und S. Rosman, Anal. Biochem. 58 (1974), 571–577] – aufgenommen und während 30 Sekunden gemischt. Die Fluoreszenz dieser Lösung wird bei einer Anregungswellenlänge von 385 nm und der Emissionswellenlänge 480 nm gemessen.

**Patentansprüche**

1. Verfahren zur Gewinnung zell- und geweberegenerierender Stoffe der Glykolipidreihe aus Blut oder Organhomogenisaten von Säugetieren, dadurch gekennzeichnet, dass man das Blut oder Organhomogenisat zuerst mit einem kurzkettigen Alkohol und einem Bakteriostatikum versetzt und dann einer Autolyse während einer Zeitspanne von mehreren Stunden bis einigen Tagen bei einer Temperatur von 4 °C bis Raumtemperatur und eine Dialyse gegen ein Gemisch von Wasser und einem niederen aliphatischen Alkohol unterwirft, derart, dass die Stoffe mit einem Molekulargewicht unterhalb 10 000 sich in dem Dialysat befinden.

2. Verfahren nach Anspruch 1, dadurch gekennzeichnet, dass man die Autolyse und Dialyse

gleichzeitig und kontinuierlich, im Gegenstromverfahren durchführt.

3. Verfahren nach Anspruch 1 oder 2, dadurch gekennzeichnet, dass man als Ausgangsmaterial Rinder- oder Kälberblut, vorzugsweise letzteres, verwendet.

4. Verfahren nach Anspruch 1, 2 oder 3, dadurch gekennzeichnet, dass man vom erhaltenen Dialysat die Glykolipide durch Zugabe von Äthanol und Stehenlassen der gebildeten äthanolischen Lösung bei einer Temperatur im Bereich von –15 bis –25 °C ausfällt und abtrennt.

5. Verfahren nach Anspruch 1, 2 oder 3, dadurch gekennzeichnet, dass man das erhaltene Dialysat durch Zugabe eines mit Wasser nicht oder nur begrenzt mischbaren Lösungsmittels oder Lösungsmittelgemisches in feste/flüssige oder flüssige/flüssige Phasen auftrennt, wobei die Glykolipide in die feste Phase bzw. in die organische, weniger polare Phase übergehen, die die Glykolipide enthaltende Phase abtrennt und allenfalls die organische Phase unter schonenden Bedingungen trocknet.

6. Verfahren nach Anspruch 5, dadurch gekennzeichnet, dass als organische Lösungsmittel zur Auftrennung des Dialysates in feste/flüssige oder flüssige/flüssige Phasen solche Äther, Kohlenwasserstoffe, halogenierte Kohlenwasserstoffe, Alkohole, Carbonsäureester und deren Gemische verwendet werden, welche mit Wasser nicht oder nur begrenzt mischbar sind.

## Claims

1. Process for the preparation of cell- and tissueregenerating substances of the glycolipid series from blood or organ homogenates from mammals, characterised in that the blood or organ homogenate is first treated with a shortchain alcohol and a bacteriostatic agent and thereafter subjected to autolysis for a period of several hours to some days at a temperature of from 4 °C to room temperature, and dialysis against a mixture of water and a lower aliphatic alcohol, in such way that the substances with a molecular weight below 10 000 are present in the dialysate.

2. Process according to Claim 1, characterised in that the autolysis and dialysis are carried out simultaneously and continuously, in a countercurrent process.

3. Process according to Claim 1 or 2, characterised in that bovine or calf blood, preferably the latter, is used as the starting material.

4. Process according to Claim 1, 2, or 3, characterised in that the glycolipids are precipitated from the resulting dialysate by addition of ethanol and leaving the ethanolic solution formed to stand at a temperature in the range from –15 to –25 °C, and are separated off.

5. Process according to Claim 1, 2 or 3, characterised in that the resulting dialysate is separated into solid/liquid or liquid/liquid phases by addition of a solvent or solvent mixture which is waterimmiscible or watermiscible only to a limited extent, the glycolipids passing into the solid phase or into the organic, less polar phase, the phase containing the glycolipids is separated off, and in case of the organic phase, the latter is dried under mild conditions.

6. Process according to Claim 5, characterised in that anyone or any mixture of ethers, hydrocarbons, halogenated hydrocarbons, alcohols and carboxylic acid esters, which are water-immiscible or water-miscible only to a limited extent is used as the organic solvent for separating the dialysate into solid/liquid or liquid/liquid phases.

## Revendications

1. Procédé d'obtention de composés de la série des glycolipides, exerçant un effet régénérateur sur les cellules et les tissues, à partir de sang ou d'homogénats d'organes de mammifères, caractérisé en ce que l'on traite d'abord le sang ou l'homogénat d'organe par un alcohol à chaîne courte et un agent bactériostatique, puis on soumet à une autolyse pendant un laps de temps de plusieurs heures à quelques jours, à une température allant de 4 °C à la température ordinaire, et à une dialyse en présence d'un mélange d'eau et d'un alcohol aliphatique inférieur, de telle sorte que les composés d'un poids moléculaire inférieur à 10 000 se trouvent dans le dialysat.

2. Procédé selon la revendication 1, caractérisé en ce que l'on effectue l'autolyse et la dialyse simultanément et de manière continue, à contrecourant.

3. Procédé selon la revendication 1 ou 2, caractérisé en ce que l'on utilise comme matériau de départ du sang de bœuf ou de veau, de préférence ce dernier.

4. Procédé selon la revendication 1, 2 ou 3, caractérisé en ce que l'on précipite les glycolipides, à partir du dialysat obtenu, par adjonction d'alcool et abandon de la solution éthanolique formée à une température dans le domaine de –15 à –25 °C, et on les sépare.

5. Procédé selon la revendication 1, 2 ou 3, caractérisé en ce que l'on sépare le dialysat obtenu, par adjonction d'un solvant ou d'un mélange de solvants non miscible à l'eau ou miscible à l'eau seulement en proportion limitée, en phases solide/liquide ou liquide/liquide, les glycolipides passant dans la phase solide ou dans la phase organique moins polaire, on sépare la phase qui contient les glycolipides et l'on sèche la phase organique, si c'est le cas, en des conditions ménagées.

6. Procédé selon la revendication 5, caractérisé en ce que l'on utilise comme solvant organique, pour séparer le dialysat en phases solide/liquide ou liquide/liquide, des éthers, hydrocarbures, hydrocarbures halogénés, alcools, esters carboxyliques ou leurs mélanges qui sont non miscibles à l'eau ou miscibles à l'eau seulement en proportion limitée.